# EUROPEAN PATENT SPECIFICATION

(11) **EP 3 897 531 B1**
(45) Date of publication and mention of the grant of the patent: **09.07.2025**
(21) Application number: 19806272.1
(22) Date of filing: 26.11.2019
(51) Int. Cl.: A61K 8/26, A61K 8/34, A61Q 11/00, A61Q 15/00, A61Q 17/00

(54) **ANTIMICROBIAL COMPOSITIONS COMPRISING MODIFIED CLAY AND BIPHENOL**
ANTIMIKROBIELLE ZUSAMMENSETZUNGEN MIT MODIFIZIERTEM TON UND BISPHENOL
COMPOSITIONS ANTIMICROBIENNES COMPRENANT DE L'ARGILE MODIFIÉE ET DU BIPHÉNOL

(30) Priority: 21.12.2018 EP 18215311
(43) Date of publication of application: 27.10.2021
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: DASGUPTA, Anindya, Bangalore 560 066 (IN); KUMARAN, Srikala, Bangalore 560 066 (IN); SAJI, Maya, Treesa, Bangalore 560 066 (IN)
(74) Representative: Tansley, Sally Elizabeth
(86) International application number: PCT/EP2019/082587
(87) International publication number: WO 2020/126351

(56) References cited:
- WO-A1-01/82922
- WO-A1-2011/036031
- WO-A1-2014/102032
- WO-A1-2017/106763

## Description

The present invention is in the field of personal care. In particular, the invention relates to antimicrobial composition that can be used, for instance, in oral care. The antimicrobial compositions comprise modified clay comprising an antimicrobial compound, and a further biphenol component. The invention further relates to uses of the antimicrobial composition.

### Background

Hygiene and appropriate personal care can prevent and treat a number of diseases caused by micro-organisms. An useful instrument to facilitate and improve hygiene includes personal care compositions. The most effective personal care compositions serving this purpose include antimicrobial components that can decrease the microbial count on skin and mucosa surfaces. For instance, antimicrobial compositions in deodorants reduce or prevent the growth of microorganisms that generate malodor or that promote the decomposition of body oils into odiferous fatty acids. Similar principles apply to shampoos, shower gels, tooth pastes, hand and body washes and other examples of personal care compositions comprising antimicrobial compositions.

The antimicrobial activity of such personal care products ensure not only a cosmetic effect but also can be an effective protection and treatment of a number of diseases and conditions affecting the skin and other body surfaces, such as acne and skin infections (e.g. eczema).

One of the most important aspects of personal care is oral health. Cavities, tartar, gum diseases e.g. gingivitis, plaque and halitosis are diseases (or conditions resulting from or that may lead to diseases) in the oral cavity that can be easily prevented or treated by adequate oral hygiene. These diseases and conditions are not only of importance from a medical perspective but also from the cosmetic point of view as they may impair one's smile appearance (such as teeth yellowing or bad breath, that not necessarily has a pathological origin). In any case, an unhealthy and/or impaired oral condition may affect people's social activities, sometimes resulting in reclusion, anxiety, depression or panic. As much as it is relevant for the oral health of human subjects, oral care is also of increasing importance in the veterinary industry, either for pets or large animals.

Oral diseases and cosmetically impairing oral conditions develop from the formation of biofilm on the surface of teeth. Oral biofilm affects the cosmetic appearance for it facilitates teeth discoloration and/or staining. Also, oral biofilm allows microorganisms attachment to the teeth surface possibly resulting in diseases.

Oral biofilm is formed by attachment of acquired pellicle, which is a thin protein-containing film derived from salivary glycoproteins, to a clean tooth surface. The pellicle allows bacteria adhesion from so-called pioneer species, such as *Actinomyces spp, Streptococcus spp, Haemophilus spp, Capnocytophaga spp, Veillonella spp,* and *Neisseria,* to the tooth surface. This early stage of biofilm formation can be easily reversed. However, if not removed, the pioneer bacteria will enable the subsequent attachment of bacterial species such as *Fusobacterium nucleatum, Treponema spp, Tannerella forsythensis, P. gingivalis, Aggregatibacter actinomycetemcomitans,* etc., that are related to the formation of plaque, tartar, gum diseases, etc.

Halitosis may be a symptom of oral diseases as it often indicates the presence of pathogens causing e.g. cavities and gingivitis. However, halitosis is not always connected to a medical condition. Bad breath, or mouth malodor, may be caused, for instance, by some medicaments, excessive caffeine consumption or reduced saliva production (dry mouth). When non pathological, halitosis can be prevented or controlled with oral care compositions, such as mouthwashes, chewing gums, toothpastes, breath tablets, and other products that ensure oral cleaning and provide a fresh breath.

WO2001/82922 describes oral compositions comprising an effective amount of a polyphenol herbal extract selected from the group consisting of magnolol, honokiol, tetrahydromagnolol, tetrahydrohonokiol, and mixtures thereof; an effective amount of a buffering agent; from about 40 percent to about 99 percent of one or more aqueous carriers; wherein the oral composition has a total water content of from about 5 percent to about 70 percent.

WO2013/081626 describes oral care compositions comprising isobutyl magnolol and propylene glycol monolaurate, for use in the treatment of caries, gingivitis, periodontitis, tooth yellowing and halitosis. Example 1 describes a composition comprising 1.4% magnolol and propylene glycol laurate that has shown superior antibacterial activity *in vitro.*

US2006/0127329 describes an antiplaque, anticalculus, and antigingivitis oral composition comprising an effective amount of an antibacterial ingredient comprising at least one active compound from an extract of magnolia, as well as an effective amount of an anticalculus system comprising tetrasodium pyrophosphate (TSPP) and sodium tripolyphosphate (STPP). The TSPP is present about 0.5 to about 2.5% and the STPP is present about 1 to about 10% based on the total weight of the composition. Tables I and II describe dentifrice compositions comprising 0.3% magnolia cortex extract in combination with 8% and 5% of the anticalculus system, respectively.

WO 2011/036031 A1 describes a bipolar antimicrobial particle for use in laundry detergent compositions, fabric conditioners, personal care and cosmetic compositions and a process for making the same. The antimicrobial particle described therein comprises a modified clay particle with an antimicrobial group attached thereto. The examples describe the preparation of the modified clay particles and its antimicrobial activity, in particular on fabrics.

There remains a need for antimicrobial compositions with further improved antimicrobial activity, in particular for use in personal care antimicrobial compositions such as compositions for oral care.

### Summary of the Invention

The present inventors have surprisingly found that modified clay particles comprising antimicrobial components present an improved antimicrobial activity when used in a composition further comprising a biphenol component.

Accordingly, in a first aspect, the invention relates to an antimicrobial composition comprising antimicrobial composition comprising:
a) a modified clay particle comprising an antimicrobial compound, the modified clay particle comprising:
   (i) an asymmetric 1:1 or 2:1:1 clay particle comprising alternating tetrahedral and octahedral sheets terminating with a tetrahedral sheet at one external surface plane and an octahedral sheet at another external surface plane, and
   (ii) at least one orally acceptable antimicrobial compound selected from a quaternary ammonium material wherein the at least one antimicrobial compound is attached to the coordinating cation on an external surface plane of the clay particle; and
b) at least one biphenol compound:
   wherein the at least one antimicrobial compound is cetylpyridinium chloride (CPC).

In a second aspect, the antimicrobial composition according to the invention is for use in the prevention or treatment of a disease of a mammal.

In a third aspect, the antimicrobial composition of the invention is for use in the removal of oral biofilm in a mammal subject.

In a fourth aspect, the invention relates to a non-therapeutic method of treating the skin or oral cavity of a mammal, the method including the step of contacting the skin or oral cavity of a mammal with the antimicrobial composition according to the present invention.

In yet another aspect, the invention relates to the use of an antimicrobial composition of the invention for reducing body malodor, preferably mouth malodor, and/or for reducing oral biofilm formation and/or for reducing tooth discoloration, in a mammal.

### Detailed Description of the Invention

### Antimicrobial particle

The composition according to the invention comprises a modified clay particle comprising an antimicrobial compound, the modified clay particle comprising an asymmetric 1:1 or 2:1:1 clay particle having alternating tetrahedral and an octahedral sheets terminating with a tetrahedral and an octahedral sheet at exterior surface planes. Particle of 1:1 clay is preferred.

The particle is prepared from a precursor with bipolar topospecific characteristics. Any chemical reaction or series of reactions wherein an antimicrobial compound is attached selectively to coordinating cations on the exterior plane of either the tetrahedral or the octahedral surface plane of asymmetric clay can be used to prepare the bipolar particle comprising an antimicrobial compound used in the present invention. In order to obtain a true bipolar antimicrobial particle it is preferred that the reaction is selective to only one of the exterior planes. By selective is meant that more than 50% of the total antimicrobial compound is present on one of the exterior planes, preferably more than 75%, more preferably than 80%, still more preferably than 90%, even more preferably than 95%, or even more than 99%. The bipolar particle comprising an antimicrobial compound used in the composition according to the invention can be prepared, e.g. by the process described in WO 2011/036031.

According to the present invention, preferred 1:1 clays include kaolinite and serpentine subgroups of minerals. The species included within the kaolinite subgroup include but are not limited to kaolinite, dickite, halloysite and nacrite. The species within the serpentine subgroup include but are not limited to chrysolite, lizardite, and amesite.

According to the present invention, preferred 2:1:1 clays include chlorite group of minerals. Chlorite is sometimes wrongly referred to as 2:2 clay by some mineralogists. The chlorite comprises tetrahedral-octahedral-tetrahedral sheets like 2:1 clays, with an extra weakly bound brucite like layer between tetrahedral layers.

The tetrahedral sheet preferably comprises coordinating tetrahedral cations of silicon.

The tetrahedral sheet may also comprise isomorphously substituted coordinating tetrahedral cations which are not silicon. Isomorphously substituted coordinating tetrahedral cations include, but are not limited to, cations of aluminum, iron or boron.

The octahedral sheet preferably comprises coordinating octahedral cation of aluminum.

The octahedral sheet may also comprise isomorphously substituted coordinating octahedral cations which are not aluminium. Isomorphously substituted coordinating octahedral cations include cations of magnesium or iron.

The at least one antimicrobial compound is attached to the coordinating cation on an external surface plane of the clay particle. Preferably, no antimicrobial compound is attached to coordination cations of non-exterior tetrahedral or octahedral plane or on the interior side of the surface sheets.

The antimicrobial compound may be attached to coordinating cations on the exterior side of the tetrahedral sheet or to the coordinating cations on the exterior side of the octahedral sheet. Preferably, the at least one antimicrobial compound is attached to the coordinating cations on the external surface of the octahedral surface plane.

The at least one antimicrobial compound may be attached to coordinating cations on the exterior side of the same surface sheet or on the exterior side of each of the tetrahedral and the octahedral surface sheets. The antimicrobial compounds may be the same or different. Preferably, the antimicrobial compound attached to the coordinating cations on the exterior side of the tetrahedral surface sheet is preferably not identical to the compound attached to the coordinating cations on the exterior side of the octahedral surface sheet.

Preferably, the modified clay particle has a clay:antimicrobial compound ratio is between 1:0.001 and 1:0.1, more preferably between 1:0.01 and 1:0.05, most preferably about 1:0.018.

### Antimicrobial compound

The antimicrobial compound is selected from the group of quaternary ammonium salts, and cetylpyridinium chloride (CPC).

The concentration of the antimicrobial compound in the antimicrobial composition will depend on the use of the composition. Accordingly, in some embodiments, the antimicrobial composition will comprise modified clay particles comprising the antimicrobial compound in a therapeutically effective amount. In another embodiments, the antimicrobial composition will comprise modified clay particles comprising the antimicrobial compound in a cosmetically effective amount.

Typically, the composition comprises from 0.001wt.% to 50 wt.% of the modified particle (*i.e.,* the particle comprising the at least one antimicrobial compound), by total weight of the composition, more preferably 0.01 wt.% to 20 wt.%, even more preferably from 0.1 wt.% to 10 wt.%, most preferably from 1wt.% to 5 wt.%, by total weight of the composition, even most preferably 0.2% to 3% by weight of the composition. Preferably, the antimicrobial compound is CPC, thus the particle is a CPC-clay particle, present in the composition in an amount between 0.01wt.% to 50 wt.%, more preferably between 0.1 wt.% to 20 wt.%, even more preferably between 1 wt.% to 5 wt.%.

### Biphenol

The antimicrobial composition according to the present invention comprises at least one biphenol. Examples of preferred at least one biphenol that is suitable for use in the present invention include 2,4'-biphenol (IUPAC name: 2,4'-Dihydroxybiphenyl), 2,2'-biphenol (2,2'-Dihydroxybiphenyl), 3,3'-biphenol (3,3'-Dihydroxybiphenyl), 4,4'-biphenol (4,4'-Dihydroxybiphenyl), and mixtures thereof. Preferably the at least one biphenol is selected from 2,4'-biphenol or 2,2'-biphenol.

Preferably, the at least one biphenol is a diallylbiphenol. This means the biphenol comprises diallyl substitution. The diallyl substitution may be present at any of the positions on biphenol structure. The preferred diallylbiphenol is a (5-3'-diallyl)biphenol or a (5-5'-diallyl)biphenol. The most preferred diallylbiphenol is a (5-3'-diallyl)biphenol.

Preferably, the at least one biphenol compound is selected from honokiol and magnolol. More preferably, the antimicrobial composition comprises two biphenol compounds, even more preferably honokiol and magnolol.

Preferably, the composition comprises biphenol in an amount from 0.001wt.% to 10wt.%, based on total weight of the composition, more preferably from 0.01% to 5%, even more preferably from 0.1 wt.% to 3 wt.%, yet more preferably from 1 wt.% to 2 wt.%, by weight of the antimicrobial composition.

In one embodiment, the antimicrobial compound used is honokiol and magnolol, and the sum of honokiol and magnolol present in the antimicrobial composition amounts to 0.01wt.% to 10 wt.%, by total weight of the composition, more preferably 0.1wt.% to 5 wt.%, even more preferably 0.5 wt.% to 2 wt.%, by total weight of the composition. Preferably, this amount of the combination of honokiol and magnolol is used in a composition comprising a modified particle which is a CPC-clay particle.

The combination of honokiol and magnolol may be present in a ratio between 30:70 to 70:30, more preferably 60:40 to 40:60, most preferably 50:50.

### Antimicrobial compositions

The antimicrobial composition according to the invention is preferably an antimicrobial composition for personal care, such as a composition selected from oral care compositions, shampoos, deodorants (optionally comprising propellant), hand- or body-wash, etc.

In one embodiment of the invention, the composition is present in creams (including UV-A and UV-B sunscreens), soaps, geals, deodorants, shampoos or hand- or body-wash compositions. Body-wash compositions are also known as detergent compositions. Deodorants, shampoos or hand- or body-wash compositions may comprise further conventional components, such as surfactants, humectants, fragrance, buffering agents, skin soothing agents, preservatives, UV-A or UV-B suncreens, etc.

Preferably, the composition comprises solvents such as ethyl alcohol, isopropanol, acetone, ethylene glycol monoethyl ether, diethylene glycol monobutyl ether, diethylene glycol monoethyl ether and mixtures thereof.

The composition may also comprise a non-phenolic alcohol. The non-phenolic alcohol preferably selected from e.g. aliphatic alcohol, monohydric alcohol. The most preferred non-phenolic alcohol is monohydric alcohol. Preferably, the alcohol having straight or branched chain of carbon atoms preferably containing from 1 to 16, more preferably from 2 to 10, even more preferably from 3 to 8 carbon atoms. Illustrative examples of alcohol that may be used in the composition include methyl alcohol, ethyl alcohol, isopropyl alcohol, n-propyl alcohol, isobutyl alcohol, n-butyl alcohol, n-pentyl alcohol, n-hexyl alcohol and mixtures thereof. Preferably, alcohol is selected from ethyl alcohol, isopropyl alcohol and mixtures thereof. Preferably, the composition comprises from 40 to 95%, more preferably from 45 to 90%, even more preferably from 50 to 85%, still more preferably 55 to 80%, yet more preferably from 60 to 75% and most preferably from 65 to 70%, by weight, of a non-phenolic alcohol.

Advantageously, the composition may preferably comprise ingredients like vitamins, anti-acne actives, anti-wrinkle, anti-skin atrophy and skin repair actives, skin barrier repair actives, non-steroidal cosmetic soothing actives, artificial tanning agents and accelerators, sebum stimulators, sebum inhibitors, anti-oxidants, protease inhibitors, skin tightening agents, anti-itch ingredients, hair growth inhibitors, 5-alpha reductase inhibitors, desquamating enzyme enhancers, anti-glycation agents and mixtures thereof.

The composition may preferably comprise powders like e.g. chalk, talc, fillers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

Preferably, the antimicrobial composition according to the invention further comprises surfactants. Preferably, the surfactants are selected from anionic, nonionic, cationic, amphoteric surfactants and mixtures thereof. More preferably, surfactants are selected from cationic surfactant, nonionic surfactant and mixtures thereof.

Preferred anionic surfactants include soap, alkyl ether sulfate and sulfonates, alkyl sulfates and sulfonates, alkylbenzene sulfonates, alkyl and dialkyl sulfosuccinates, C8-C20 acyl isethionates, acyl glutamates, C8-C20 alkyl ether phosphates and mixtures thereof.

Preferred nonionic surfactants are those with a C10-C20 fatty alcohol or acid hydrophobe condensed with from 2 to 100 moles of ethylene oxide or propylene 30 oxide per mole of hydrophobe; C2-C10 alkyl phenols condensed with from 2 to 20 moles of alkylene oxide; mono- and di- fatty acid esters of ethylene glycol; fatty acid monoglyceride; sorbitan, mono- and di- C8-C20 fatty acids; block copolymers (ethylene oxide/propylene oxide); polyoxyethylene sorbitan and mixtures thereof. Alkyl polyglycosides and saccharide fatty amides (e.g. methyl gluconamides) are also suitable nonionic surfactants.

Preferred cationic surfactant include cetyl trimethylammonium bromide, benzalkonium halides that are also also known as alkyldimethylbenzylammonium halides. Preferred cationic surfactant that may be used in the composition is benzalkonium chloride, also known as alkyldimethylbenzylammonium chloride.

Preferred amphoteric surfactant include amide, betaine and amine oxide type. Particularly amphoteric surfactants include cocodiethanol amide and cocomonoethanol amide, cocoamidopropyl betaine and coco amido propyl amine oxide. A preferred amphoteric surfactant that may be used as a surfactant in the composition is cocoamidopropyl betaine.

When incorporated in the composition, surfactants may be present in an amount from 1 to 80%, preferably from 3 to 60%, more preferably from 5 to 40%, even more preferably from 7 to 30%, further more preferably from 9 to 15 % by weight of the composition.

Preferably, the composition further comprises a cosmetically or pharmaceutically acceptable base. The cosmetically or pharmaceutically acceptable base is preferably in the form of a cream, lotion, gel or emulsion. Water and/or alcohol may also be used a cosmetically or pharmaceutically acceptable base. Alcohol may be a mono or polyhydric alcohol. Monohydric alcohols often are short chain, by which is meant that they contain up to 6 carbons, and in practice is most often ethanol or sometimes iso-propanol. Polyhydric alcohols commonly comprise ethylene or propylene glycol, or a homologue can be employed such as diethylene glycol. Preferably, the cosmetically or pharmaceutically acceptable base is present preferably from 10 to 99.9%, more preferably from 50 to 99%, even more preferably from 60 to 85% and further more preferably from 65 to 80% by weight of the composition.

In a preferred embodiment, the antimicrobial composition is present in an oral care product. Accordingly, the composition according to the invention is preferably included in an oral care product selected from toothpaste, dentifrice, tooth powder, topical oral gel, mouth rinse, denture product, mouth spray, lozenge, oral tablet, chewing gum, impregnated dental implement, dental floss, and combinations thereof. More preferably, the oral care product is dental care product, such as tooth powder, toothpaste, impregnated dental implement, dental floss.

Preferably, the composition comprises one or more orally acceptable component, such as abrasives, orally accepted actives (such as Fluor), teeth whiteners, fragrances, stabilizers, preservatives, among other conventional components to oral care compositions.

### Uses of the antimicrobial composition

The present inventors have found that the antimicrobial composition according to the invention has an improved antimicrobial activity. Accordingly, the antimicrobial composition can be used for cosmetic (non-therapeutic) or therapeutic applications.

### a) Therapeutic uses

In one aspect, the antimicrobial composition according to the invention is for use in the prevention or treatment of a disease of a mammal. Diseases as used herein refer to diseases caused by micro-organisms on body surfaces (skin, nails, mucosa, etc.). In one embodiment the diseases are skin diseases such as selected from the group comprising acne, eczema, or bacterial or fungal infections on the scalp, nails or skin, such as those caused by *Propionibacteria spp., Corynebacteria spp., Actinobacteriales, Staphylococci spp.* (e.g. *S. epidermidis), Lactobacilales, Clostridiales, proteobacteria, Flavobacteriales, Bacteriodales, Malassezia* yeasts (e.g. *Malassezia furfur* and *Malassezia lobose*)*,* among others.

Preferably, the use is in the prevention or treatment of a disease in the oral cavity. Preferred diseases in the oral cavity are selected from caries, tartar, dental plaque, gum diseases, and combinations thereof, for instance diseases caused by *Actinomyces spp, Streptococcus spp* (such as *S*. *mutans* and *S. sobrinus*)*, Lactobacillus ssp.* (such as, *Lactobacillus acidophilus), Nocardia spp., Haemophilus spp, Capnocytophaga spp, Veillonella spp, Neisseria, Fusobacterium,* such as *F. nucleatum, Treponema spp, Tannerella ssp.,* such as *T. forsythensis, P. gingivalis, Aggregatibacter actinomycetemcomitans,* and others.

In a further aspect, the antimicrobial composition of the invention is for use in the removal of oral biofilm in a mammal subject.

Typically, the particle comprising an antimicrobial compound is preferably incorporated in the composition for the cosmetic use in an amount from 0.05% to 10% by weight of the composition, more preferably from 0.1% to 10%, most preferably from 0.2 % to 5 % by weight of the composition. The clay:antimicrobial ratio is between 1:0.001 and 1:0.1, more preferably between 1:0.01 and 1:0.05, most preferably about 1:0.018. Preferably, the antimicrobial composition for the therapeutic uses according to the invention comprises a therapeutically effective amount of an antimicrobial compound.

### b) Cosmetic/Non-therapeutic uses

The present invention provides cosmetic uses of the antimicrobial composition on body surfaces, such as skin, teeth, scalp, mucosa (gums), tongue, etc. The inventors have found an improved antimicrobial activity on micro-organisms normally found on said body surfaces and that may impair body appearance (such as teeth yellowing) or body odor, in particular mouth malodor. Preferably, the antimicrobial composition is for the cosmetic use in the prevention or treatment of halitosis caused by microorganisms in the oral cavity.

Accordingly, in yet another aspect, the invention relates to a non-therapeutic method of treating the skin or oral cavity of a mammal, the method including the step of contacting the skin or oral cavity of a mammal with the antimicrobial composition according to the present invention.

In another aspect, the invention relates to the use of an antimicrobial composition of the invention for reducing body malodor, preferably mouth malodor, and/or for reducing oral biofilm formation and/or for reducing tooth discoloration, in a mammal.

The particle comprising an antimicrobial compound is preferably incorporated in the composition for the cosmetic use in an amount from 0.05% to 10% by weight of the composition, more preferably from 0.1% to 10%, most preferably from 0.2 % to 5 % by weight of the composition.

### Examples

### Example 1: Preparation of modified clay particle

To prepare the modified clay particle comprising an antimicrobial compound according to the invention, the following method was used:
5g of Kaolinite (Super shine 90, EICL) was taken in a 500ml of 0.1N HCl (Merck) solution and sonicated for 30 minutes. The pH is then increased to 9 by addition of NaOH (Merck) solution drop wise. To this 10g CPC was added and the suspension was stirred over a magnetic stirrer (Spinpot) for 6 hours while maintaining the temperature of the solution at 75-80 °C. The suspension was then washed with water for about 10 times to remove the excess CPC and a final ethanol (Les Alcools De Commerce) wash was given. The clay was then dried in a hot air oven.

To determine that CPC was attached on the clay after reaction FTIR- spectroscopy method was utilized. The instrument used was Perkin Elmer instruments, Spectrum One FT-IR Spectrometer. Powder (diffuse reflectance) technique was utilized for this measurement. Clay as control and reacted clay of the invention were grounded with 50% w/w of KBr in a pestle and mortar and then IR was done on these powders. The IR spectrum of the reacted clay was compared against that of pure clay. New peaks were observed in the reacted clay at the wave numbers of 2926 cm-1, 2855 cm-1, 1487 cm-1 and 1466 cm-1. The peaks at 2926 cm-1 and 2855 cm-1 are due to the C-C stretching of the alkyl chain of the CPC, while the peaks at 1487 cm-1 and 1466 cm-1 are due to the ring carbon and nitrogen of the CPC.

### Example 2: Antimicrobial activity

Antimicrobial activity of the compositions according to the invention has been compared with compositions comprising either only the modified clay particle comprising an antimicrobial compound ('CPC', prepared as in Ex. 1), or only biphenol compounds ('HM', *i.e.*, Honokiol and Magnolol, each 98% pure from World Way Biotech, China). The compositions (Table 1) were tested according to the following method.

Pellicle coated HAP disc was treated with toothpaste base formulation topped up with actives for 2 mins, subjected to sterile water wash and challenged with salivary flora and incubated overnight. At the end of incubation, the discs were stained with Plaque disclosing dye (Plak-check, Vishal Dentocare PVT.LTD). The detailed method is as follows:
Early morning saliva was collected from 5-6 volunteers and pooled. The pooled saliva was pelleted by centrifuging at 4000g for 10min to produce salivary pellicle (the supernatant was filter sterilized through 0.22um filter and stored at 4°C) and salivary flora (pellet was washed twice in 5ml of 1XPBS (pH7.0) by centrifugation). The salivary flora was adjusted to 108 Log CFU/ml (0.2 OD620nm) in ultra-filtered Tryptone yeast extract broth (nutrient media) containing 2% sucrose.

Each sterile HAP was coated with salivary pellicle (discs were placed horizontally in 12 well plate) to allow the initial pellicle formation on the sterile HAP surface. The pellicle coated HAP disc was dip washed and challenged with 30% diluted toothpaste slurry topped up with actives for 2 mins. After 2 min of contact time, the treated HAP was dip washed thrice consecutively in 5ml of sterile water. The treated HAP discs were challenged with salivary flora and incubated anaerobically at 37°C for 22-24h.

At the end 22-24h incubation, the disc was dip washed thrice in of sterile distilled water. The rinsed disc was immersed in plaque disclosing solution (Plak-Check containing erythrosin, from Vishal Dentocare Pvt Ltd) for 5 min. At the end of 5 min, the stained disc was dip washed thrice consecutively in 5ml of sterile distilled water. The stained discs were dried at 25+ 3°C for 1 hour. Once dried the stain on the discs was eluted using 1ml of 0.1N NaOH. The absorbance of eluted solutions was read at OD540nm. Higher the absorbance reading, more the formed biofilm on the disc. The results are described in table 1 below.

**Table 1**

| | % Biofilm |
|---|---|
| Base toothpaste | 100 |
| 0.33% HM | 97 |
| 0.5% CPC-Clay | 89 |
| 0.33% HM + 0.5% CPC-Clay | 34 |

### Example 3: Malodor reduction

Solutions of clay with antimicrobial compound at different concentrations were prepared as described in Example 1 (concentrations of solutions given in Table 2 below). Salivary thiols were quantified using BDC-OH colorimetric assay [4,4'-bis (dimethyl amino) diphenyl carbinol], where blue BDC-OH decolorises upon reation with thiol group.

Human saliva was collected and pooled from volunteers. All the saliva samples were added test solutions and kept in incubation at 37 °C for 24h in a shaker incubator. After spinning, the supernatant was separated for analysis by adding 50µl of supernatant to 250µl BDC-OH solution (TCI Chemicals) in a 96 well plate, which was covered with foil and incubated for 1h at 37 °C. Standard solutions of BDC-OH were also prepared.

Samples were read OD at 630nm using a plate reader. The calibration curve of the standards against the OD readings of generated for the test samples was plotted. Using the calibration curve equation, the amount of BDC-OH remaining in the test wells from the OD readings was calculated. The results are shown in Table 2.

**Table 2**

| | Amount of BDC-OH remaining (mM) |
|---|---|
| Water control | 0.0 |
| 0.005% CPC-Clay | 0.64 |
| 0.005% HM | 4.78 |
| 0.005% CPC-Clay + 0.005% HM | 8.18 |

## Claims

1. An antimicrobial composition comprising:
a) a modified clay particle comprising an antimicrobial compound, the modified clay particle comprising:
(i) an asymmetric 1:1 or 2:1:1 clay particle comprising alternating tetrahedral and octahedral sheets terminating with a tetrahedral sheet at one external surface plane and an octahedral sheet at another external surface plane, and
(ii) at least one orally acceptable antimicrobial compound selected from a quaternary ammonium material, wherein the at least one antimicrobial compound is attached to the coordinating cation on an external surface plane of the clay particle; and
b) at least one biphenol compound.
wherein the at least one antimicrobial compound is cetylpyridinium chloride (CPC).

2. The composition according to claim 1, wherein the at least one antimicrobial compound is attached to coordinating cations on the external surface of the octahedral surface plane.

3. The composition according to claim 1 or 2, wherein clay:antimicrobial ratio is of between 1:0.001 to 1:0.1.

4. The composition according to any of the preceding claims, wherein the antimicrobial particle is present in an amount between 0.01-5 wt.%, by weight of the composition.

5. The composition according to claim 1, wherein the at least one biphenol compound is selected from 2,4'-biphenol, 2,2'-biphenol, 3,3'-biphenol, 4,4'-biphenol or mixtures thereof, preferably 2,4'-biphenol or 2,2'-biphenol.

6. A composition according to claim 5, wherein the biphenol compound is a di-allylbiphenol compound.

7. A composition according to 6, wherein the di-allylbiphenol compound is a (5-3'diallyl)biphenol or a (5-5'-diallyl)biphenol.

8. A composition according to claim 7, wherein the biphenol is selected from honokiol or magnolol, preferably honokiol.

9. A composition according to any one of the preceding claims, wherein the composition comprises one or more pharmaceutically acceptable components.

10. The composition according to anyone of claims 1 to 9, wherein the composition is included in an oral care product selected from toothpaste, dentifrice, tooth powder, topical oral gel, mouth rinse, denture product, mouth spray, lozenge, oral tablet, chewing gum, impregnated dental implement, dental floss, and combinations thereof.

11. An antimicrobial composition, according to any of claims 1 to 10 , for use in the prevention or treatment of a disease of a mammal, preferably for use in the prevention or treatment of a disease in the oral cavity selected from the group comprising caries, tartar, dental plaque, gum diseases, and combinations thereof.

12. An antimicrobial composition, according any of claims 1 to 10, for use in the removal of oral biofilm in a mammal subject.

13. A non-therapeutic method of treating the skin and/or oral cavity of a mammal, the method including the step of contacting the skin and/or oral cavity of a mammal with the antimicrobial composition according to any of claims 1 to 10.

14. A non-therapeutic use of an antimicrobial composition according to any one of claims 1 to 10, for reducing body malodor, preferably mouth malodor, and/or for reducing oral biofilm formation and/or for reducing tooth discoloration, in a mammal.

## Patentansprüche

1. Antimikrobielle Zusammensetzung, umfassend:
a) ein modifiziertes Tonpartikel, das eine antimikrobielle Verbindung umfasst, wobei das modifizierte Tonpartikel umfasst:
(i) ein asymmetrisches 1:1- oder 2:1:1-Tonpartikel, das alternierend tetraedrische und oktaedrische Schichten umfasst, die mit einer tetraedrischen Schicht auf einer äußeren Oberflächenebene und einer oktaedrischen Schicht auf einer anderen äußeren Oberflächenebene enden, und
(ii) mindestens eine oral verträgliche antimikrobielle Verbindung, ausgewählt unter einem quaternären Ammoniummaterial, wobei die mindestens eine antimikrobielle Verbindung an das koordinierende Kation auf einer äußeren Oberflächenebene des Tonpartikels gebunden ist; und
b) mindestens eine Biphenolverbindung,
wobei die mindestens eine antimikrobielle Verbindung Cetylpyridiniumchlorid (CPC) ist.

2. Zusammensetzung nach Anspruch 1, wobei die mindestens eine antimikrobielle Verbindung an koordinierende Kationen auf der Außenfläche der oktaedrischen Oberflächenebene gebunden ist.

3. Zusammensetzung nach Anspruch 1 oder 2, wobei das Verhältnis Ton :
antimikrobielle Verbindung zwischen 1:0,001 bis 1:0,1 liegt.

4. Zusammensetzung nach irgendeinem der vorhergehenden Ansprüche, wobei das antimikrobielle Partikel in einer Menge zwischen 0,01-5 Gew.-%, bezogen auf das Gewicht der Zusammensetzung, vorliegt.

5. Zusammensetzung nach Anspruch 1, wobei die mindestens eine Biphenolverbindung unter 2,4'-Biphenol, 2,2'-Biphenol, 3,3'-Biphenol, 4,4'-Biphenol oder Mischungen davon, vorzugsweise 2,4'-Biphenol oder 2,2'-Biphenol, ausgewählt ist.

6. Zusammensetzung nach Anspruch 5, wobei die Biphenolverbindung eine Diallylbiphenolverbindung ist.

7. Zusammensetzung nach Anspruch 6, wobei die Diallylbiphenolverbindung ein (5-3'-Diallyl)biphenol oder ein (5-5'-Diallyl)biphenol ist.

8. Zusammensetzung nach Anspruch 7, wobei das Biphenol unter Honokiol oder Magnolol, vorzugsweise Honokiol, ausgewählt ist.

9. Zusammensetzung nach irgendeinem der vorhergehenden Ansprüche, wobei die Zusammensetzung eine oder mehrere pharmazeutisch verträgliche Komponenten umfasst.

10. Zusammensetzung nach irgendeinem der Ansprüche 1 bis 9, wobei die Zusammensetzung in einem Mundpflegeprodukt enthalten ist, das unter Zahnpasta, Zahnputzmittel, Zahnpulver, topischem Mundgel, Mundspülmittel, Zahnprothesenprodukt, Mundspray, Lutschtabletten, Mundtablette, Kaugummi, imprägniertem Dentalimplantat, Zahnseite und Kombinationen davon, ausgewählt ist.

11. Antimikrobielle Zusammensetzung nach einem der Ansprüche 1 bis 10 zur Verwendung bei der Vorbeugung oder Behandlung einer Erkrankung eines Säugetiers, vorzugsweise zur Verwendung bei der Vorbeugung und Behandlung einer Erkrankung in der Mundhöhle, ausgewählt aus der Karies, Zahnstein, Zahnbelag, Zahnfleischerkrankungen und Kombinationen davon umfassenden Gruppe.

12. Antimikrobielle Zusammensetzung nach einem der Ansprüche 1 bis 15 zur Verwendung bei der Entfernung von oralem Biofilm bei einem Säugetier.

13. Nicht-therapeutisches Verfahren zur Behandlung der Haut und/oder der Mundhöhle eines Säugetiers, wobei das Verfahren den Schritt des Inkontaktbringens der Haut und/oder der Mundhöhle eines Säugetiers mit der antimikrobiellen Zusammensetzung nach einem der Ansprüche 1 bis 10 umfasst.

14. Nicht-therapeutische Verwendung einer antimikrobiellen Zusammensetzung nach irgendeinem der Ansprüche 1 bis 10 zur Verringerung der Bildung von Körpergeruch, vorzugsweise Mundgeruch, und/oder zur Verringerung der Bildung eines oralen Biofilms und/oder zur Verringerung von Zahnverfärbungen bei einem Säugetier.

## Revendications

1. Composition antimicrobienne comprenant:
a) une particule d'argile modifiée contenant un composé antimicrobien, la particule d'argile modifiée comprenant:
(i) une particule d'argile asymétrique 1:1 ou 2:1:1 comprenant des feuillets tétraédriques et octaédriques alternés se terminant par un feuillet tétraédrique au niveau d'un plan de surface externe et un feuillet octaédrique au niveau d'un autre plan de surface externe, et
(ii) au moins un composé antimicrobien oralement acceptable choisi parmi un produit d'ammonium quaternaire, où le au moins un composé antimicrobien est lié au cation de coordination sur un plan de surface externe de la particule d'argile; et
b) au moins un composé biphénol,
où le au moins un composé antimicrobien est le chlorure de cétylpyridinium (CPC).

2. Composition selon la revendication 1, où le au moins un composé antimicrobien est lié à des cations de coordination sur la surface externe du plan de surface octaédrique.

3. Composition selon la revendication 1 ou 2, où le rapport argile:antimicrobien est entre 1:0,001 et 1:0,1.

4. Composition selon l'une quelconque des revendications précédentes, où la particule antimicrobienne est présente en une quantité entre 0,01 et 5 % en poids, en poids de la composition.

5. Composition selon la revendication 1, où le au moins un composé biphénol est choisi parmi le 2,4'-biphénol, le 2,2'-biphénol, le 3,3'-biphénol, le 4,4'-biphénol ou leurs mélanges, de préférence le 2,4'-biphénol ou le 2,2'-biphénol.

6. Composition selon la revendication 5, où le composé biphénol est un composé diallylbiphénol.

7. Composition selon la revendication 6, où le composé diallylbiphénol est un (5-3'-diallyl)biphénol ou un (5-5'-diallyl)biphénol.

8. Composition selon la revendication 7, où le biphénol est choisi parmi l'honokiol ou le magnolol, de préférence l'honokiol.

9. Composition selon l'une quelconque des revendications précédentes, où la composition comprend un ou plusieurs composants pharmaceutiquement acceptables.

10. Composition selon l'une quelconque des revendications 1 à 9, où la composition est incluse dans un produit de soins bucco-dentaires choisi parmi une pâte dentifrice, un dentifrice, une poudre dentifrice, un gel buccal topique, un bain de bouche, un produit pour prothèse dentaire, un spray buccal, une pastille, un comprimé buccal, un chewing-gum, un instrument dentaire imprégné, un fil dentaire et leurs combinaisons.

11. Composition antimicrobienne, selon l'une quelconque des revendications 1 à 10, destinée à être utilisée dans la prévention ou le traitement d'une maladie d'un mammifère, de préférence destinée à être utilisée dans la prévention ou le traitement d'une maladie dans la cavité buccale choisie dans le groupe comprenant les caries, le tartre, la plaque dentaire, les maladies des gencives et leurs combinaisons.

12. Composition antimicrobienne, selon l'une quelconque des revendications 1 à 10, destinée à être utilisée dans le retrait d'un biofilm buccal chez un sujet mammifère.

13. Procédé non thérapeutique de traitement de la peau et/ou de la cavité buccale d'un mammifère, le procédé incluant l'étape de mise en contact de la peau et/ou de la cavité buccale d'un mammifère avec la composition antimicrobienne selon l'une quelconque des revendications 1 à 10.

14. Utilisation non thérapeutique d'une composition antimicrobienne selon l'une quelconque des revendications 1 à 10, pour réduire les mauvaises odeurs corporelles, de préférence les mauvaises odeurs buccales, et/ou pour réduire la formation d'un biofilm buccal et/ou pour réduire la décoloration des dents, chez un mammifère.
